(19) Europäisches Patentamt
European Patent Office
Office européen des brevets

(11) Numéro de publication: **0 003 206**
**B1**

(12) # FASCICULE DE BREVET EUROPEEN

(21) Numéro de dépôt: **79420002.2**

(22) Date de dépôt: **10.01.79**

(51) Int. Cl.³: **C 07 C 67/04,**
C 07 C 69/14, C 07 C 69/63

(54) **Procédé pour la préparation d'esters.**

(30) Priorité: **16.01.78 FR 7801571**

(43) Date de publication de la demande:
**25.07.79 Bulletin 79/15**

(45) Mention de la délivrance du brevet:
**25.03.81 Bulletin 81/12**

(84) Etats Contractants Désignés:
**BE CH DE GB IT LU NL SE**

(56) Documents cités:
**US - A - 3 474 131**
**US - A - 2 858 331**

(73) Titulaire: **RHONE-POULENC INDUSTRIES**
**22, avenue Montaigne**
**F-75008 Paris (FR)**

(72) Inventeur: **Gruffaz, Max**
**6, Avenue du Général de Gaulle**
**F-69350 La Mulatiere (FR)**

(72) Inventeur: **Micaelli, Odile**
**11, rue Montvert**
**F-69008 Lyon (FR)**

(74) Mandataire: **Rioufrays, Roger et al,**
**RHONE-POULENC INDUSTRIES Centre de Recherches des Carrières Service Brevets**
**F-69190 Saint-Fons (FR)**

**EP 0 003 206 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

# Procédé pour la préparation d'esters

La présente invention a trait à un procédé pour la préparation d'esters. Elle a plus spécialement pour objet un procédé de préparation d'esters d'éthyle ou d'isopropyle, par réaction d'un acide carboxylique sur l'oléfine correspondante.

Depuis de nombreuses années, il est connu que les acides carboxyliques s'additionnent sur les oléfines, en présence de catalyseurs acides. Si les avantages de cette méthode de synthèse d'esters, par rapport aux procédés conventionnels, faisant intervenir en phase liquide des alcools et des catalyseurs acides minéraux, ont été reconnus rapidement, cette méthode, notamment dans le cas où l'on cherche à obtenir des esters d'éthyle est restée une curiosité de laboratoire, en raison du manque d'efficacité des catalyseurs acides, employés jusqu'alors.

En effet, il ressort de travaux antérieurs (Ind. & Eng. CHEM 1951, 43, p. 1596—1600—et J. Appl. Chem. 1963, p. 544—547) que l'éthylène en particulier, ne peut être estérifié par un acide carboxylique, dans des conditions relativement sévères qu'en présence de grandes quantités de catalyseurs acides. Ces conditions extrêmement dures favorisent les réactions secondaires, en particulier les réactions de polymérisation, qui se produisent au détriment de l'ester recherché.

Un but de la présente invention est de fournir des catalyseurs plus efficaces pour la préparation d'esters, par réaction de l'éthylène ou du propylène sur des acides carboxyliques.

Les recherches qui ont abouti à la présente invention ont montré que l'on peut obtenir des esters d'éthyle ou d'isopropyle, avec un rendement acceptable, tout en opérant avec des quantités moindres de catalyseurs, en faisant réagir en phase liquide, de l'éthylène ou du propylène avec au moins un acide carboxylique, en présence d'au moins un acide perfluoroalcanesulfonique en tant que catalyseur.

Des recherches ont montré d'une manière en soi remarquable que l'on peut faire réagir d'une manière avantageuse de l'éthylène et au moins un acide carboxylique, en phase liquide, en présence d'au moins un acide perfluoroalcanesulfonique, en quantité catalytique, pour obtenir des esters d'éthyle avec un rendement acceptable, dans des conditions relativement douces, de telle sorte que le processus puisse être appliqué plus aisément à l'échelle industrielle, et avec succès pour divers acides carboxyliques.

Il a donc maintenant été trouvé que les acides perfluoroalcanesulfoniques de formule

$$C_nF_{2n+1}SO_3H$$

dans laquelle n est un nombre entier compris entre 1 et 8 ($1 \leqslant n \leqslant 8$), sont des catalyseurs efficaces de la réaction d'estérification de l'éthylène ou du propylène par les acides carboxyliques en phase liquide.

L'invention envisage également l'utilisation, comme catalyseur de ces acides, partiellement ou totalement sous la forme de leurs esters d'alcoyles inférieurs, ayant notamment de 1 à 4 atomes de carbone dans le radical alcoyle, et plus particulièrement, partiellement ou totalement, sous forme de leurs esters d'éthyle ou d'isopropyle.

De préférence on opère avec l'acide trifluorométhanesulfonique, et/ou ses esters d'alcoyles ayant notamment de 1 à 4 atomes de carbone dans le radical alcoyle, en raison notamment de sa plus grande stabilité dans les conditions opératoires.

L'acide trifluorométhanesulfonique et/ou son ester d'éthyle ou d'isopropyle convient particulièrement bien à la mise en oeuvre de la présente invention.

Le choix de l'acide carboxylique à mettre en oeuvre, selon la présente invention, dépendra bien évidemment de la nature de l'ester d'éthyle ou d'isopropyle recherché.

A titre d'exemples d'acides carboxyliques utilisables dans le cadre de la présente invention, on peut citer:

les monoacides aliphatiques ayant jusqu'à 20 atomes de carbone dans la molécule, saturés ou insaturés, pouvant porter des substituants, notamment un ou plusieurs atomes d'halogènes, en particulier les acides acétique, bromacétique, chloracétique, propionique, $\alpha$-bromo- ou $\alpha$-chloropropioniques, isobutyrique, hexanoïque, acrylique et méthacrylique. L'invention envisage également les monoacides aromatiques éventuellement substitués, en particulier par un ou plusieurs atomes d'halogènes, notamment les acides benzoïque et toluiques, les acides alicycliques tels les acides naphténiques, les diacides aliphatiques ayant de 3 à 6 atomes de carbone dans la molécule, notamment les acides succinique et adipique, et les diacides aromatiques, notamment les acides phtaliques.

Les monoacides aliphatiques saturés ayant au plus 10 atomes de carbone dans la molécule et substitués par un atome de brome ou de chlore en $\alpha$, ou par plusieurs atomes de brome et/ou de chlore constituent une classe particulièrement intéressante à mettre en oeuvre dans le cadre de la présente invention. De préférence, on utilise un acide choisi dans le groupe constitué par l'acide monochloracétique, l'acide dichloracétique, l'acide monobromacétique, l'acide dibromacétique, l'acide $\alpha$-chloropropionique, l'acide $\alpha$-bromopropionique, et l'acide $\alpha,\alpha$-dichloropropionique.

La quantité de catalyseur à introduire dans le milieu réactionnel peut être aussi faible que 0,05 mole par litre de mélange réactionnel et, en général, il n'est pas utile de dépasser 1 mole

par litre de mélange. De préférence, cette quantité est compris entre 0,1 et 0,5 mole par litre de mélange réactionnel.

Selon le procédé de l'invention, on obtient des esters d'éthyle ou d'isopropyle, par réaction en phase liquide, dans un milieu essentiellement anhydre, de l'éthylène ou du propylène sous pression, sur un acide carboxylique en présence d'au moins un acide perfluoroalcanesulfonique comme catalyseur.

Lorsque l'on fait réagir le propylène, selon le procédé de l'invention, une pression légèrement supérieure à la pression atmosphérique, et de préférence au plus égale à 10 bars est suffisante, la température de réaction pouvant varier entre 80 et 120°C.

Lorsque l'on fait réagir l'éthylène, selon le procédé de l'invention, une pression comprise entre 20 et 60 bars convient particulièrement bien, la température de réaction pouvant varier entre 100 et 200°C, et de préférence entre 130 et 160°C.

Les exemples ci-après illustrent la mise en oeuvre du procédé selon l'invention:

Exemples 1 à 4 = Préparation d'esters d'ethyle

Dans une bombe de 250 cm³ en Hastelloy C, on charge l'acide carboxylique et le catalyseur. On porte à la température choisie dans un four à agitation longitudinale puis on envoie de l'éthylène sous pression, la pression étant de 40 bars. On arrête la réaction au temps désiré.

Les résultats obtenus, exprimés par le taux de transformation (TT) de l'acide en ester d'éthyle recherché, ainsi que les conditions particulières sont données dans le tableau ci-après.

Le sélectivité en ester recherché est de l'ordre de 100%. A titre de comparaison, les essais a et b sont réalisés avec l'acide sulfurique comme catalyseur.

| N° Exemple | Catalyseur | | Acide Carboxylique | | T°C | Duree en Heures | TT % |
|---|---|---|---|---|---|---|---|
| | Nature | Mole/Litre | Nature | Mole | | | |
| 1 | $CF_3SO_3H$ | 0,324 | $CH_3COOH$ | 1,64 | 150 | 4 | 9,3 |
| a | $H_2SO_4$ | 0,925 | $CH_3COOH$ | 1,64 | 150 | 4 | 3,5 |
| 2 | $CF_3SO_3H$ | 0,323 | $CH_2Cl{-}COOH$ | 1,40 | 140 | 3 | 64,2 |
| b | $H_2SO_4$ | 0,963 | $CH_2Cl\ COOH$ | 1,40 | 150 | 3 | 56,5 |
| 3 | $C_6F_{13}SO_3H$ | 0,1 | $CH_2Cl{-}COOH$ | 1,41 | 150 | 4 | 31,1 |
| 4 | $CF_3SO_3H$ | 0,113 | $CH_3CHCl{-}COOH$ | 1,18 | 150 | 4 | 20,0 |

Exemple 5 = Préparation de l'acétate d'isopropyle

Selon la procédure générale décrite pour les exemples précédents, on charge:

1,66 mole d'acide acétique

0,21 mole/litre d'acide trifluorométhanesulfonique.

Le mélange est porté à 100°C. On envoie alors du propylène sous une pression de 10 bars. Au bout de quatre heures, on arrête la réaction.

Le taux de transformation de l'acide acétique est de 88,7%, la sélectivité en acétate d'isopropyle est de l'ordre de 100%.

**Revendications**

1. Procédé de préparation d'esters d'éthyle ou d'isopropyle, par réaction en phase liquide, de l'éthylène ou du propylène, sous pression, sur un acide carboxylique caractérisé en ce que l'on opère en présence de 0,05 à 1 Mole par litre de milieu réactionnel, d'au moins un acide de formule:

$$C_nF_{2n+1}SO_3H$$

dans laquelle n est un entier compris entre 1 et 8 ($1 \leqslant n \leqslant 8$), éventuellement, partiellement ou totalement sous la forme d'un ester d'alcoyle inférieur, comme catalyseur.

2. Procédé de préparation d'esters d'isopropyle en phase liquide, par réaction du propylène, sous une pression au plus égale à 10 bars, sur un acide carboxylique, à une température comprise entre 80 et 120°C, en présence de 0,05 à 1 Mole par litre de milieu réactionnel d'au moins un acide de formule:

$$C_nF_{2n+1}SO_3H$$

dans laquelle n est un entier compris entre 1 et 8 ($1 \leqslant n \leqslant 8$).

3. Procédé de préparation d'esters d'éthyle, en phase liquide, par réaction de l'éthylène sous une pression comprise entre 20 et 60 bars, sur un acide carboxylique, à une température comprise entre 100 et 200°C, en présence de 0,05 à 1 Mole par litre de milieu réactionnel d'au moins un acide de formule:

$$C_nF_{2n+1}SO_3H$$

dans laquelle n est un entier compris entre 1 et 8 ($1 \leqslant n \leqslant 8$).

4. Procédé selon l'une quelconque des revendications précédentes caractérisées en ce que l'acide carboxylique mis en oeuvre est l'acide acétique.

5. Procédé selon l'une quelconque des revendications précédentes caractérisé en ce que l'on opère en présence d'acide trifluorométhanesulfonique comme catalyseur.

6. Procédé selon l'une quelconque des revendications précédentes caractérisé en ce que le catalyseur est partiellement ou totalement sous forme d'un ester d'alcoyle ayant de 1 à 4 atomes de carbone dans le radical alcoyle.

7. Procédé selon l'une quelconque des revendications précédentes, caractérisé en ce que le catalyseur est totalement ou partiellement sous forme d'ester d'éthyle ou d'isopropyle.

8. Procédé selon l'une quelconque des revendications précédentes, caractérisé en ce que la quantité de catalyseur est comprise entre 0,1 et 0,5 mole par litre de mélange réactionnel.

9. Procédé selon l'une quelconque des revendications précédentes caractérisé en ce que l'acide carboxylique mis en oeuvre est choisi dans le groupe constitué par l'acide monochloracétique, l'acide monobromacétique, l'acide $\alpha$-chloropropionique et l'acide $\alpha$-bromopropionique.

**Patentansprüche**

1. Verfahren zur Herstellung von Äthyl- oder Isopropylestern mittels Umsetzung in flüssiger Phase von Äthylen oder Propylen unter Druck mit einer Carbonsäure, dadurch gekennzeichnet, daß man in Gegenwart von 0,05 bis 1 Mol je Liter Reaktionsmedium mindestens einer Säure der allgemeinen Formel

$$C_nF_{2n+1}SO_3H,$$

in der n eine ganze Zahl von 1 bis 8 ist ($1 \leqslant n \leqslant 8$), gegebenenfalls teilweise oder ganz in Form eines niederen Alkylesters, als Katalysator, arbeitet.

2. Verfahren zur Herstellung von Isopropylestern in flüssiger Phase durch Umsetzung von Propylen unter einem Druck von höchstens 10 bar mit einer Carbonsäure bei einer Temperatur von 80 bis 120°C in Gegenwart von 0,05 bis 1 Mol je Liter Reaktionsmedium mindestens einer Säure der allgemeinen Formel

$$C_nF_{2n+1}SO_3H,$$

in der n eine ganze Zahl von 1 bis 8 ist ($1 \leqslant n \leqslant 8$).

3. Verfahren zur Herstellung von Äthylestern in flüssiger Phase mittels Umsetzung von Äthylen unter einem Druck von 20 bis 60 bar mit einer Carbonsäure bei einer Temperatur von 100 bis 200°C in Gegenwart von 0,05 bis 1 Mol je Liter Reaktionsmedium mindestens einer Säure der allgemeinen Formel

$$C_nF_{2n+1}SO_3H,$$

in der n eine ganze Zahl von 1 bis 8 ist ($1 \leqslant n \leqslant 8$).

4. Verfahren nach irgendeinem der vorangehenden Ansprüche, dadurch gekennzeichnet, daß die eingesetzte Carbonsäure Essigsäure ist.

5. Verfahren nach irgendeinem der vorangehenden Ansprüche, dadurch gekennzeichnet, daß man in Gegenwart von Trifluormethansulfonsäure als Katalysator arbeitet.

6. Verfahren nach irgendeinem der vorangehenden Ansprüche, dadurch gekennzeichnet, daß der Katalysator teilweise oder ganz in Form eines Alkylesters mit 1 bis 4 Kohlenstoffatomen in der Alkylgruppe vorliegt.

7. Verfahren nach irgendeinem der vorangehenden Ansprüche, dadurch gekennzeichnet, daß der Katalysator vollständig oder teilweise in Form des Äthylesters oder des Isopropylesters vorliegt.

8. Verfahren nach irgendeinem der vorangehenden Ansprüche, dadurch gekennzeichnet, daß die Menge des Katalysators 0,1 bis 0,5 Mol je Liter Reaktionsgemisch ausmacht.

9. Verfahren nach irgendeinem der vorangehenden Ansprüche, dadurch gekennzeichnet, daß die eingesetzte Carbonsäure ausgewählt wird aus der Gruppe, bestehend aus Monochloressigsäure, Monobromessigsäure, $\alpha$-Chlorpropionsäure und $\alpha$-Brompropionsäure.

**Claims**

1. Process for the preparation of ethyl or isopropyl esters by reacting, in the liquid phase, ethylene or propylene, under pressure, and a carboxylic acid, characterised in that the reaction is carried out in the presence of 0.05 to 1 mol, per litre of reaction medium, of at least one acid of the formula:

$$C_nF_{2n+1}SO_3H$$

in which n is an integer between 1 and 8 ($1 \leq n \leq 8$), optionally partially or totally in the form of a lower alkyl ester, as a catalyst.

2. Process for the preparation of isopropyl esters, in the liquid phase, by reacting propylene, under a pressure equal to at most 10 bars, with a carboxylic acid, at a temperature between 80 and 120°C, in the presence of 0.05 to 1 mol, per litre of reaction medium, of at least one acid of the formula:

$$C_nF_{2n+1}SO_3H$$

in which n is an integer between 1 and 8 ($1 \leq n \leq 8$).

3. Process for the preparation of ethyl esters, in the liquid phase, by reacting ethylene, under a pressure between 20 and 60 bars, with a carboxylic acid, at a temperature between 100 and 200°C, in the presence of 0.05 and 1 mol, per litre of reaction medium, of at least one acid of the formula:

$$C_nF_{2n+1}SO_3H$$

in which n is an integer between 1 and 8 ($1 \leq n \leq 8$).

4. Process according to any one of the preceding claims, characterised in that the carboxylic acid used is acetic acid.

5. Process according to any one of the preceding claims, characterised in that the reaction is carried out in the presence of trifluoromethanesulphonic acid as the catalyst.

6. Process according to any one of the preceding claims, characterised in that the catalyst is partially or totally in the form of an alkyl ester having from 1 to 4 carbon atoms in the alkyl radical.

7. Process according to any one of the preceding claims, characterised in that the catalyst is totally or partially in the form of the ethyl or isopropyl ester.

8. Process according to any one of the preceding claims, characterised in that the amount of catalyst is between 0.1 and 0.5 mol per litre of reaction mixture.

9. Process according to any one of the preceding claims, characterised in that the carboxylic acid used is chosen from the group comprising monochloroacetic acid, monobromoacetic acid, $\alpha$-chloropropionic acid and $\alpha$-bromopropionic acid.